(19) Europäisches Patentamt

European Patent Office

Office européen des brevets

(11) Veröffentlichungsnummer: **0 252 409**
**A2**

# (12) EUROPÄISCHE PATENTANMELDUNG

(21) Anmeldenummer: **87109365.4**

(22) Anmeldetag: **30.06.87**

(51) Int. Cl.⁴: **C12N 15/00** , C12P 21/02 , C12N 13/00

(30) Priorität: **10.07.86 DE 3623194**

(43) Veröffentlichungstag der Anmeldung:
**13.01.88 Patentblatt 88/02**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Wolfrum, Juergen, Prof. Dr.**
**Suedring 2**
**D-3405 Rosdorf 2(DE)**
Erfinder: **Greulich, Karl Otto, Dr.**
**Ploeck 25-27**
**D-6900 Heidelberg(DE)**
Erfinder: **Weber, Gerd, Dr.**
**Gregor-Mendel-Strasse 22**
**D-6802 Ladenburg(DE)**
Erfinder: **Zimmermann, Karin, Dr.**
**Kirschenallee 7/1**
**D-7102 Weinsberg(DE)**
Erfinder: **Butz, Peter**
**Langgewann**
**D-6900 Heidelberg(DE)**
Erfinder: **Wiegand, Rosemarie**
**Quinkestrasse 8**
**D-6900 Heidelberg(DE)**
Erfinder: **Monajembashi, Shamci**
**Rahmengasse 5**
**D-6900 Heidelberg(DE)**

(54) **Verfahren zur Fusion biologischer Zellen und Protoplasten mit einem Laser-Mikrostrahl.**

(57) Verfahren zur Fusion biologischer Zellen und Protoplasten mit Hilfe eines in der Wellenlänge frei abstimmbaren Laser-Mikrostrahls, dadurch gekennzeichnet, daß durch in ein Mikroskop eingekoppelte, fokussierte Laserpulse, die in der Objektebene Pulsenergien von 0,1 bis 100 • $10^{-6}$ J/Puls, vorzugsweise von 1 bis 50 • $10^{-6}$ J/Puls haben, biologische Zellen und Protoplasten zur Fusion gebracht werden.

EP 0 252 409 A2

## Verfahren zur Fusion biologischer Zellen und Protoplasten mit einem Laser-Mikrostrahl

Die vorliegende Erfindung betrifft ein Verfahren zum Fusionieren von biologischen Zellen und Protoplasten beliebiger Herkunft mit Hilfe eines in der Wellenlänge frei abstimmbaren Laser-Mikrostrahls. Es betrifft sowohl adhärent wachsende Zellen als auch Zellen in Suspension.

Es ist aus der Publikation Devel. Biol. 101 (1984) 240-245, bekannt, daß sich in Frühstadien der Entwicklung eines Eies des Wurms Canhaerabditis elegans Zellen innerhalb des Eies, die in sehr engem Kontakt miteinander stehen, fusionieren lassen in dem Sinne, daß die gemeinsame Zellwand mit einem Laser-Mikrostrahl zerschossen wird und damit die Cytoplasmen benachbarter Zellen ineinanderfließen.

Ein sehr viel häufiger anstehendes Problem der Biologie und Biotechnologie ist jedoch die Fusion von einzelnen Zellen in Suspension, also mit vergleichsweise geringer Intensität der Zellkontakte sowie der Fusion von adhärent wachsenden Zellen.

Hierzu ist bekannt, daß sich bestimmte biologische Zellen miteinander fusionieren lassen, wenn eine Zellsuspension fusogene Agentien, wie z.B. Polyethylenglycol (PEC), DEAE Dextran oder inaktivierten Sendai Virus enthält (Planta 115 (1974) 355-367). Diese, heute als Standardverfahren anerkannten Fusionsverfahren erlauben zwar Massenproduktion von Zellhybriden, lassen aber keine selektive Fusion von einzelnen Zellen unter Sichtbeobachtung zu. Außerdem sind die Fusionsagentien Zellgifte. Eine erheblich verbesserte Möglichkeit zur Beobachtung und selektiven Fusion bietet die Elektrofusion (Bioch. Bioph. Acta 694 (1982) 227-277 und Bioch. Soc: Transactions 14 (1986) 246-249), ein Verfahren, bei dem Zellen durch Dielektrophorese mit einem Wechselfeld zwischen Elektroden gesammelt und durch einen Feldpuls von einigen 100 V/cm zur Fusion gebracht werden. Elektrofusion ist für eine Reihe von Zelltypen schwer durchführbar, da in unphysiologischen Medien gearbeitet wird. Die Elektrofusion kann nicht berührungsfrei durchgeführt werden. Ferner entstehen bei Elektrofusion häufig Fusionsprodukte aus mehr als zwei Zellen, was für eine Reihe von Anwendungen nicht vorteilhaft ist. Hierzu ist weiterhin bekannt, daß die Anzahl unerwünschter Hybride bei der Herstellung von Hybridomen reduziert werden kann, wenn man die Reaktionspartner chemisch verkoppelt (Nature 310 (1984) 732-734).

Der vorliegenden Erfindung lag nun die Aufgabe zugrunde, Zellen unter physiologischen Bedingungen zu fusionieren und insbesondere berührungs frei Zellhybride aus definitiv zwei Zellen herzustellen. Dies sollte unter Sichtbeobachtung geschehen, damit z.B. besonders geeignete Zellen für die Fusion verwendet werden können oder Zellpaare aus einem Gemisch mehrerer Zelltypen ausgesucht werden können.

Die Aufgabe wurde erfindungsgemäß dadurch gelöst, daß Pulse eines Lasers in ein Mikroskop eingekoppelt wurden, und diese bis auf die Wellenlänge des verwendeten Lichtes focussierten Pulse auf biologische Zellen oder Protoplasten gerichtet wurden. Dabei stellte sich überraschenderweise heraus, daß bei Pulsenergien von 0,1 bis 100 • 10-6 J/Puls keine irreversiblen Läsionen auftreten, sondern daß die Zellmembranen von aneinanderliegenden Zellen beginnen, sich zu vereinigen und einen Hybriden zu bilden. Nachdem der Fusionsvorgang durch Laserpulse gestartet worden ist, läuft er selbständig weiter, bis der Hybrid abgerundet ist und das Erscheinungsbild der Ausgangszellen, aber mit größerem Volumen, annimmt. Die Zeit von der Anregung des Fusionsvorgangs durch Laserpulse bis zur Abrundung wird als Abrundungszeit bezeichnet und beträgt je nach Zelltyp einige Sekunden bis einige Minuten. Die so gebildeten Hybride können je nach Zielsetzung des Experiments auf verschiedene Arten separiert werden, z.B. durch Selektion in einem Medium, das den Hybriden Wachstumsvorteile bietet, oder durch Aufnehmen der Hybride in feine Glaskapillaren mit Hilfe eines Mikromanipulators.

Vorteilhafte Ausgestaltungen des erfindungsgemäßen Verfahrens sind Gegenstand der Unteransprüche 2 bis 6.

Das erfindungsgemäße Verfahren basiert im wesentlichen auf folgenden Voraussetzungen:

1. Es wird ein in der Wellenlänge abstimmbarer Laser-Mikrostrahl zur Fusion biologischer Zellen eingesetzt.

2. Der Laserstrahl ist bis auf Beugungsbegrenzung fokussiert, d.h. je nach Wellenlänge des verwendeten Lichts auf 0.2 bis 0.8 μm genau lokalisierbar.

Mit der Erfindung wurde es zum ersten Male möglich, ausgesuchte Zellpaare unter Sichtbeobachtung berührungsfrei in physiologischer Umgebung zu fusionieren ohne Substanzen zu verwenden, die als Zellgifte wirken können.

### Beispiel 1 (Fusion von Hefezellen)

Hefezellen (Bäckerhefe, ca. 10 % in Wasser, auf Objektträger leicht eingeengt) wurden in wäßrigen Suspensionen aufgenommen und auf einen Objektträger gebracht. Mit Hilfe von 1 bis 10 Einzelpulsen von 3 • 10−6 J/Puls (am Objektrevol-

ver des Mikroskops gemessen) wurden ganze Zellgruppen oder ausgewählte Zellpaare zur Fusion gebracht. Die verwendete Wellenlänge des Laserlichts war 343 nm. Die Abrundungszeit betrug ca. 10 sec. Diese Fusion soll der gezielten genetischen Veränderung von Hefezellen unter Sichtbeobachtung dienen, die in technischen Prozessen, z.B. beim Brauen, eingesetzt werden sollen.

### Beispiel 2 (Fusion von Raps-Protoplasten)

Rapsprotoplasten wurden in isotoner Lösung aufgenommen und auf einen Objektträger gebracht. Benachbarte Protoplasten werden durch 10 bis 50 Laserpulse von 0.1 • $10^{-6}$ J/Puls zur Fusion gebracht. Die Abrundungszeit beträgt ca. 1 min. Die Vitalität des Fusionsproduktes wurde dadurch bestätigt, daß noch 30 min nach der Fusion eine ausgeprägte Cytoplasmaströmung vorlag. Die verwendete Wellenlänge betrug 339 nm.

### Beispiel 3 (Fusion von Mauszellen)

Myelom-Zellen und frisch präparierte Milzzellen von Mäusen wurden mittels Avidin-Biotin gekoppelt. Ein Tropfen der Zellsuspension wurde in eine Petrischale pipettiert. Eng beieinander liegende Zellpaare wurden mit Pulsen von $10^{-6}$ J/Puls bei einer Wellenlänge von 343 nm fusioniert.

Die fusionierten Zellen unterschieden sich nicht mehr von unbestrahlten Myelomzellen. Hieraus kann man auf die Vitalität der Zellen schließen. Die Zeitspanne des Fusionsprozesses von der Bestrahlung bis zur vollständigen Abrundung der Fusionszellen beträgt ca. 5 min.

Mit diesem Verfahren bietet sich die Möglichkeit, selektiv unter Sichtbeobachtung sowohl immunologische als auch in anderen Richtungen relevante Zellen zu fusionieren. Nach erfolgter Fusion können die Zellen leicht weiterkultiviert und entsprechend der gewünschten Zellprodukte ausgetestet werden. Aufgrund der gezielten Laserfusion wird eine langwierige Ausplattierung der Zellen unnötig und das Zeit und Material raubende Austesten nach den gewünschten Produkten gegenüber der herkömmlichen Antikörper-Produktion erheblich verkürzt. Dies könnte von wesentlicher Bedeutung sein bei der Produktion von tumorspezifischen Antikörpern, wenn diese aus therapeutischen Gründen sehr schnell bereitgestellt werden müssen.

### Beispiel 4 (menschliche Zellen und Zellen in Gewebsverbänden)

Neben den unter 1) bis 3) beschriebenen Beispielen konnten auch lymphoide menschliche Zellen und Epithelzellen der Känguruhratte in Gewebsverbänden fusioniert werden. Aus der Tatsache, daß das Spektrum der bisher erfolgreich verschmolzenen Zellen von Hefen über Pflanzenprotoplasten und menschlichen Blutzellen bis hin zu gewebebildenden Epithelzellen reicht, kann geschlossen werden, daß laserinduzierte Zellfusion universell, d.h. bei geeigneter Wahl der physikalisch-chemischen Umgebung für alle Zellfusionsprobleme einsetzbar ist.

## Ansprüche

1. Verfahren zur Fusion biologischer Zellen und Protoplasten mit Hilfe eines in der Wellenlänge frei abstimmbaren Laser-Mikrostrahls, dadurch gekennzeichnet, daß durch in ein Mikroskop eingekoppelte, fokussierte Laserpulse, die in der Objekteebene Pulsenergien von 0,1 bis 100 • $10^{-6}$ J/Puls, vorzugsweise von 1 bis 50 • $10^{-6}$ J/Puls haben, biologische Zellen und Protoplasten zur Fusion gebracht werden.

2. Verfahren nach Anspruch 1, dadurch gekennzeichnet, daß physiologische Kulturmedien verwendet werden, und weder Zellgifte noch Lösungsmittel niedriger Leitfähigkeit notwendig sind.

3. Verfahren nach Anspruch 1 und 2, dadurch gekennzeichnet, daß unter Sichtbeobachtung Zellhybride aus mindestens zwei Zellen hergestellt werden, insbesondere auch aus Zellen sehr unterschiedlicher Größe.

4. Verfahren nach Anspruch 3, dadurch gekennzeichnet, daß berührungsfrei, z.B. ohne Einsatz von Elektroden, gearbeitet wird, indem der Mikrostrahl direkt auf die Zellen gerichtet wird.

5. Verfahren nach Anspruch 1 bis 4, dadurch gekennzeichnet, daß Zellen in kompletten Geweben selektiv miteinander fusioniert werden.

6. Verfahren nach Anspruch 1 bis 4, dadurch gekennzeichnet, daß Hybride für die Produktion monoklonaler Antikörper hergestellt und ohne langwierige Selektionsverfahren verwendet werden.